# EUROPEAN PATENT APPLICATION

(11) **EP 2 005 917 A1**
(43) Date of publication of application: **24.12.2008**
(21) Application number: 08011261.8
(22) Date of filing: 20.06.2008
(51) Int. Cl.: A61F 2/06, A61F 2/86, A61F 2/90, A61F 2/88, B29C 35/08

(54) **Medical tube, medical instrument, stent set and endoscope device**

(30) Priority: 21.06.2007 JP 2007163631
(71) Applicant: Olympus Medical Systems Corp., Tokyo 151-0072 (JP)
(72) Inventor: Goto, Hiroaki, Tokyo 151-0072 (JP)
(74) Representative: von Hellfeld, Axel

(57) **Abstract**

A first aspect of the present invention relates to a medical tube (3) including: a tube main body; a sealed space (4) which is formed in the tube main body and is sealed on both ends; and a variable hardness resin (5) which fills the sealed space and the hardness of which can be changed by supplying energy thereto. A second aspect of the present invention relates to a medical tube including: a tube main body having at least one lumen; a variable hardness resin which fills the inside of the lumen and the hardness of which can be changed by supplying energy thereto; and an outflow preventing mechanism which prevents the filling variable hardness resin from flowing out from the lumen.

## Description

### BACKGROUND OF THE INVENTION

### Field of the Invention

The present invention relates to a medical instrument, such as a stent employed to dilate esophageal strictures, a medical tube composing the medical instrument, a stent set and an endoscope device for placing the medical instrument within the body.

### Description of Related Art

While devices such as esophageal stents are required to be pliable to facilitate insertion into the body, once positioned, these devices must possess a certain degree of rigidity (hardness), as well. In order to satisfy these contrary demands, Published Japanese Translation No. 2005-507707 of the PCT International Publication discloses a device in which the main part of a graft is composed of a double-walled plastic tube having an inner and an outer walls. After positioning this graft at a specific site inside the body, a filler material is injected in between the inner and outer walls, thereby maintaining the graft in a specific form.

However, the following problems remain with the technique disclosed in Published Japanese Translation No. 2005-507707 of the PCT International Publication.

Namely, this technique calls for positioning the graft in advance inside the body, and then maintaining the graft in a particular shape by injecting a filler material in between the inner and outer walls. Accordingly, there is a concern that the filler material could overflow into the body during the injection process. Thus, there are strict limitations on the material which can be employed as the filler material, and materials that are potentially harmful to the body cannot be used at all.

### SUMMARY OF THE INVENTION

The present invention was conceived in view of the above-described circumstances, and has as its objective the provision of a medical tube which provides pliability when inserted into the body but ensures a certain degree of rigidity after insertion, and for which there is no concern about overflow of resin or other such employed filler material into the body. The present invention further relates to a medical instrument, a stent set, and an endoscope device for placing the medical instrument into the body.

The present invention employs the following means to resolve the problems described above.

In accordance with a first aspect of the present invention, a medical tube includes: a tube main body; a sealed space which is formed in the tube main body and is sealed on both ends; and a variable hardness resin which fills the sealed space and the hardness of which can be changed by supplying energy thereto.

With this medical tube, the hardness of the variable hardness resin can be changed by applying heat, UV rays or other such energy to the tube after it has been placed into the body. Accordingly, the pliability of the tube can be maintained during insertion into the body since the variable hardness resin has not yet been cured. Then, once retained in the body, the hardness of the variable hardness resin can be increased, so that a certain degree of rigidity (hardness) is obtained. Further, since the sealed space is filled with the variable hardness resin, manipulation to fill the tube with a variable hardness resin after positioning in the body is not necessary. In addition, such undesirable circumstances as outside leakage of the variable hardness resin due to carelessness during such manipulation does not occur.

In accordance with a second aspect of the present invention, a medical tube includes a tube main body having at least one lumen, a variable hardness resin which fills the inside of the lumen and the hardness of which can be changed by supplying energy thereto, and an outflow preventing mechanism which prevents the filling variable hardness resin from flowing out of the lumen.

According to this medical tube, as disclosed above, because the variable hardness resin has not yet been cured at the time of insertion into the body, the medical tube remains pliable. However, once retained inside the body, the hardness of the variable hardness resin can be increased so that a certain degree of rigidity (hardness) can be obtained. In addition, the lumen has been filled in advance with the variable hardness resin, with the outflow of resin from the lumen prevented by the outflow preventing mechanism. Thus, manipulation to fill the lumen with a variable hardness resin after placement into the body is not necessary, so that such undesirable circumstances as outside leakage of the variable hardness resin due to carelessness during such manipulation does not occur.

In the medical tube according to the present invention, it is desirable that the variable hardness resin be an energy curable resin which cures with the addition of energy.

According to this medical tube, after positioning this medical tube inside the body, for example, the medical instrument can be hardened by the application of energy.

In a medical instrument employing the medical tube according to the present invention, it is desirable that a linear member be formed of the medical tube with the energy curable resin filling along the central line of the tube main body, and that the cylindrically shaped outer form of the medical instrument be formed of the linear member.

In this medical instrument, the linear member is composed of the medical tube, and the cylindrical outer form of the medical instrument is formed of the linear member. As a result, the outer form of the medical instrument can be formed into an optional shape.

It is desirable that the cylindrical outer shape of the medical instrument according the present invention be formed by interweaving the liner member.

In this medical instrument, the cylindrical outer shape is formed by interweaving the linear member formed of medical tube, making it possible to form a medical instrument without employing a reinforcing material such as a sheet member or the like.

In the medical instrument according to the present invention, it is desirable that the cylindrical outer shape be formed by adhering the linear member to a surface of a flexible sheet.

By employing a flexible sheet in this medical instrument, it is acceptable to dispose the linear member only to the site where the rigidity of the flexible sheet is insufficient, and to then perform curing. As a result, manufacture of the medical instrument is facilitated.

In the medical instrument according to the present invention, it is desirable that the linear member be affixed to the flexible sheet in a ring or helix shape.

Because it is possible to limit the amount of the relatively expense linear member employed in this medical instrument, a corresponding reduction in costs can be achieved.

In the medical instrument according to the present invention, it is desirable that the tube main body be made of a UV transmitting material and that the energy curable resin be made of a UV curable resin.

In this medical instrument, UV rays are radiated from the outside via the tube main body. As a result, it is possible to cure the UV curable resin inside the tube, so that the resin curing operation can be carried out easily within the body.

In a medical instrument employing the medical tube according to the present invention, it is desirable that a cylindrical catheter main body be formed such that both ends of the tube main body are opened and that the sealed space is formed within the side walls of this catheter main body, with this sealed space filled with the variable hardness resin.

According to this medical instrument, the medical instrument is directly formed, without forming the linear members, which are intermediates. As a result, manufacture becomes easier.

In the medical instrument according to the present invention, it is desirable that the medical instrument is a stent.

With this medical instrument, pliability during insertion can be obtained, making it possible to place the device at the desired site inside the body, e.g., esophagus, etc. Moreover, the required rigidity (hardness) can be obtained by curing the resin following placement. As a result, it is possible to provide a stent with excellent usability which takes full advantage of the properties of the resin.

In the stent set according to the present invention, the stent, e.g., the medical instrument, is covered with packaging that does not transmit UV rays.

In this stent set, the stent is covered with packaging and thus maintained in a state impervious to UV radiation during the time from manufacture until insertion into the patient's body. As a result, such undesirable events as careless exposure to UV rays leading to curing prior to insertion into the patient's body can be avoided.

The endoscope device according to the present invention is provided with an endoscope equipped with an inserted part that incorporates the medical instrument, and a UV ray probe that can be inserted into a channel of the inserted part and which emits UV rays.

In this endoscope device, the medical instrument can be placed at a specific site inside the body using the inserted part of the endoscope, after which the medical instrument is radiated with UV rays using the UV probe. As a result, the UV curable resin which fills the inside of the medical instrument can be cured.

In the present invention, it is possible to maintain pliability during insertion into the body, since the resin is not yet cured. Moreover, following retention within the body, a certain degree of rigidity (hardness) can be obtained by varying the hardness of the resin. Further, since the tube is filled with the resin in advance, an operation to fill the tube with the resin after placement inside the body is not necessary. Accordingly, such undesirable circumstances as the overflow of resin to the outside during such an operation does not occur.

### BRIEF DESCRIPTION OF THE DRAWINGS

FIG 1 is a perspective view of a stent showing a first embodiment of the present invention.
FIG 2 is an exploded perspective view of the linear members composing the stent.
FIG 3 is a partial perspective view showing the stent set in place to the endoscope device.
FIG 4 is a cross-sectional view showing the stent being placed inside the body using the endoscope device.
FIG 5 is a cross-sectional view showing the radiation of UV rays onto the stent.
FIG 6 is a perspective view of the stent showing a second embodiment of the present invention.
FIG 7 is a perspective view of the stent showing a third embodiment of the present invention.
FIG 8 is a perspective view of the stent showing a fourth embodiment of the present invention.
FIG 9 is a perspective view of the stent showing a fifth embodiment of the present invention.
FIG 10 is a perspective view of the stent showing a sixth embodiment of the present invention.
FIG 11 is a perspective view of the stent showing a seventh embodiment of the present invention.
FIG 12 is a perspective view of the catheter showing an eighth embodiment of the present invention.
FIG 13 is a perspective view of the catheter showing a ninth embodiment of the present invention.

### PREFERRED EMBODIMENTS OF THE PRESENT INVENTION

Preferred embodiments of the present invention will now be explained with reference to the figures.

### <First Embodiment>

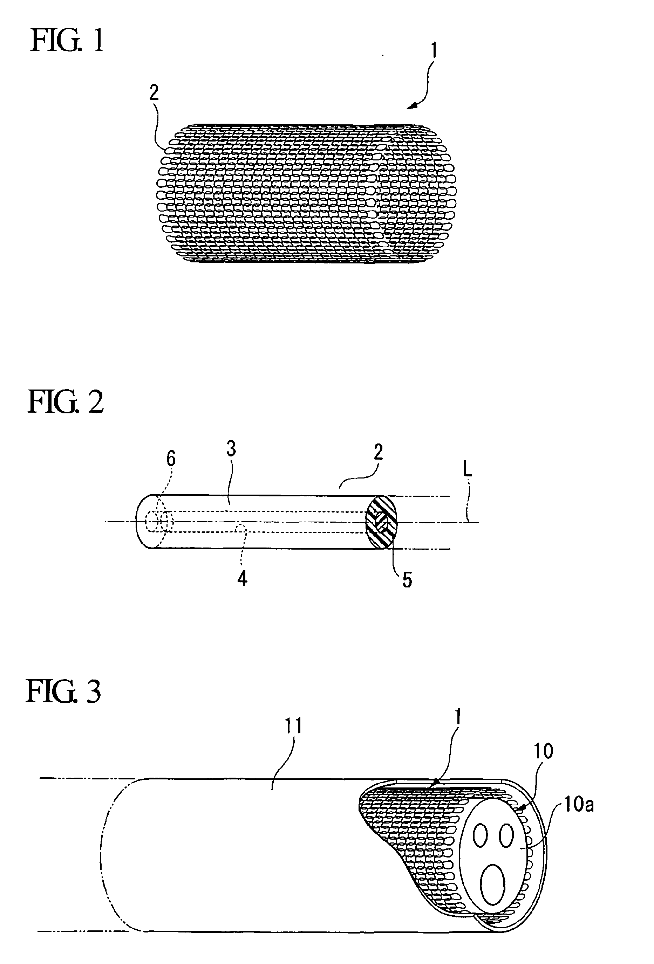

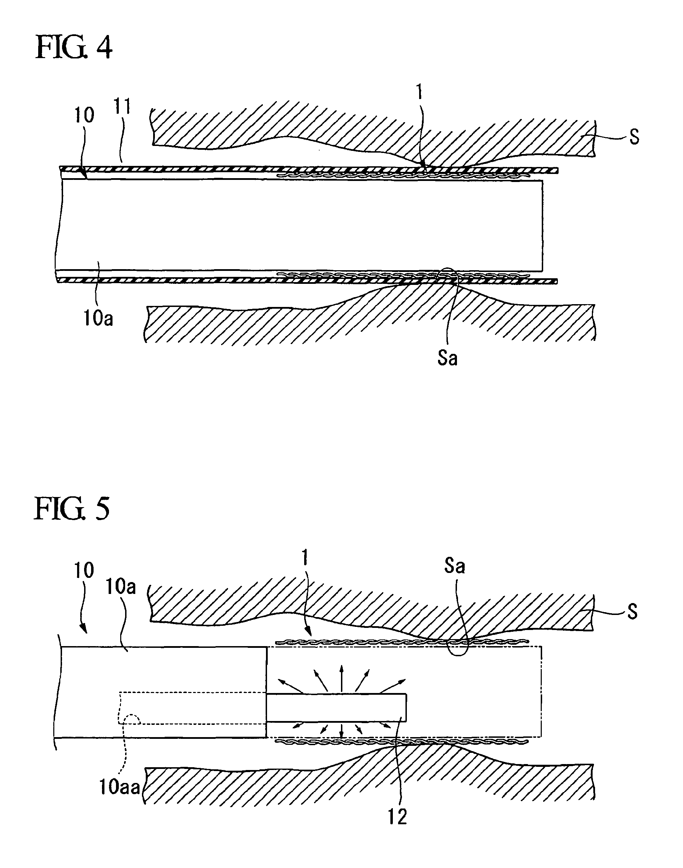

A first embodiment of the present invention is shown in FIGS. 1 through 5. FIG 1 is a perspective view of the stent. FIG 2 is an exploded perspective view of the linear members composing the stent. FIG 3 is a partial perspective view showing the stent set in place to the endoscope device. FIG 4 is a cross-sectional view showing the stent being positioned inside the body using the endoscope device. FIG 5 is a cross-sectional view showing the radiation of UV rays onto the stent.

As shown in FIG.1, the stent 1 according to this embodiment has the shape of a cylinder overall by interknitting of linear members 2. As the method for knitting linear members 2, a method such as circular knitting (horizontal knitting) may be employed, in which a looped form is supported by forming and connecting looped stitches in a helix shape, and then alternately winding together in the radial direction a looped stitch and another looped stitch adjacent thereto along the center axis. Other knitting methods are also acceptable. Moreover, it is not necessary that the cylindrical shape of the entire form be an exact circle in cross-section. Rather, it is also acceptable if the cross-sectional shape is an oval or a polygon due to properties of the linear member 2. The linear members 2 that compose the stent 1 may be a single element, or may comprise of two or more elements. When the stent 1 is knitted of a linear member 2 consisting of multiple elements, then a single type of material or two or more types of material may be employed for linear members 2.

As shown in FIG 2, a linear member 2 is formed such that there is a sealed space 4 formed along the central axis L of a medical tube 3. This sealed space 4 is filled with UV curable resin 5. The sealed space 4 may be designed to be partitioned by walls set at specific lengths along the longitudinal direction of medical tube 3, or may be continuous along the length of the tube without any partitioning walls, with each end of the tube sealed close by a plugging member 6.

The sealed space 4 need not be formed to have the same axis as the center axis of the medical tube 3; rather, it may be slightly deviated therefrom. Further, it is also acceptable to form two sealed spaces in the medical tube 3, such as the shape of a pair of eyeglasses, or to form a three or more multiple sealed spaces. In other words, any sealed space into which UV curable resin 5 can be introduced is acceptable.

The material employed for the medical tube 3 is one which can transmit UV rays and which is not harmful to the human body. Suitable examples thereof include fluorine resins, nylon resins, urethane resins and the like. Further, the UV transmissible material need not be fully transparent; a semi-transparent material is acceptable as well, provided that it permits passage of UV rays.

Placement of a stent 1 having the above-described structure at a stricture site Sa in the esophagus S will now be explained.

First, as shown in FIG 3, an overtube 11 is provided so as to surround an inserted part 10a of an endoscope 10. Stent 1 is then inserted into position between the inserted part 10a and the overtube 11. In this arrangement, the inserted part 10a of the endoscope 10 is then introduced per os together with the overtube 11, and advanced into the esophagus S. The stricture site Sa is confirmed via the endoscopic image. After confirming the position of the stricture site Sa, positioning of the overtube 11 and the inserted part 10a in which the stent 1 is set is carried out while checking using the endoscopic image, until the stent 1 reaches a position opposing the stricture site Sa as shown in FIG 4.

Next, the overtube 11 is withdrawn while confirming via the endoscopic image, leaving the stent 1 exposed within the esophagus S as shown in FIG 5. Next, with stent 1 in this position, the inserted part 10a of the endoscope 10 is retracted, and the stent 1 is retained at the position opposing the stricture site Sa.

Next, a UV probe 12 is projected out from the distal end of the channel 10aa of the inserted part 10a, and UV rays are radiated from the probe 12. The radiated UV rays pass through the medical tube 3 to reach the UV curable resin 5 which fills the inner sealed space 4, thereby curing the UV curable resin 5. As a result, the stent 1 has sufficient rigidity to withstand lateral pressure from the stricture site Sa.

When there is a concern that the shape of the stent 1 will change due to lateral pressure from the stricture site when the inserted part of the endoscope is retracted, the inserted part 10a of the endoscope may be moved a small amount relative to the stent 1 and part-by-part curing with UV irradiation from the UV probe 12 can be carried out to the portion of the stent that is pulled out from the inserted part 10a of the endoscope. In other words, by gradually pulling the inserted part 10a of the endoscope from stent 1 while UV curing the stent 1 part-by-part, the stent 1 can be maintained in the desired shape.

In the case where there are multiple channels 10aa in the endoscope, a channel other than that employed for the insertion of the UV probe 12 can be used to expose a procedural instrument from the distal end of the inserted part 10a, and this procedural instrument may be used to hold the stent 1 in a specific shape as it undergoes UV curing. It is also acceptable to employ a single channel and to then interchange the UV probe and the procedural instrument.

The method for UV curing the stent 1 is not limited to a UV probe; rather, the light source used for endoscopic visualization may be interchanged with a UV radiating bulb, and the UV rays radiated from this bulb may be employed.

Once stent 1 has been UV cured, inserted part 10a of the endoscope 10 may be withdrawn from the body along with the overtube 11.

In a stent 1 of the above-described structure, the UV curable resin 5 has not yet been cured at the time of insertion into the body and is therefore pliable. As a result, the stent 1 as a whole possesses sufficient pliability.

As a result, stent 1 can be easily positioned between the overtube 11 and the inserted part 10a of the endoscope 10. Moreover, when passing the apparatus with the stent 1 inserted into the overtube 11 through the laryngopharynx, for example, the stent 1 is able to deform suitably in a unitary manner with the overtube 11 to pass through the laryngopharynx. Once the stent 1 is retained inside the body, the UV curable resin 5 is cured, providing the stent 1 with a certain degree of rigidity (hardness) and its required holding strength.

The sealed space 4 is filled with the UV curable resin 5 in advance in the linear members 2 that compose the stent. Accordingly, an operation to fill the inside of the linear members 2 with the UV curable resin 5 after placement of the stent 1 in the body is not necessary. Thus, such undesirable circumstances as overflow of the UV curable resin to the outside due to carelessness during such an operation does not occur.

### <Second Embodiment>

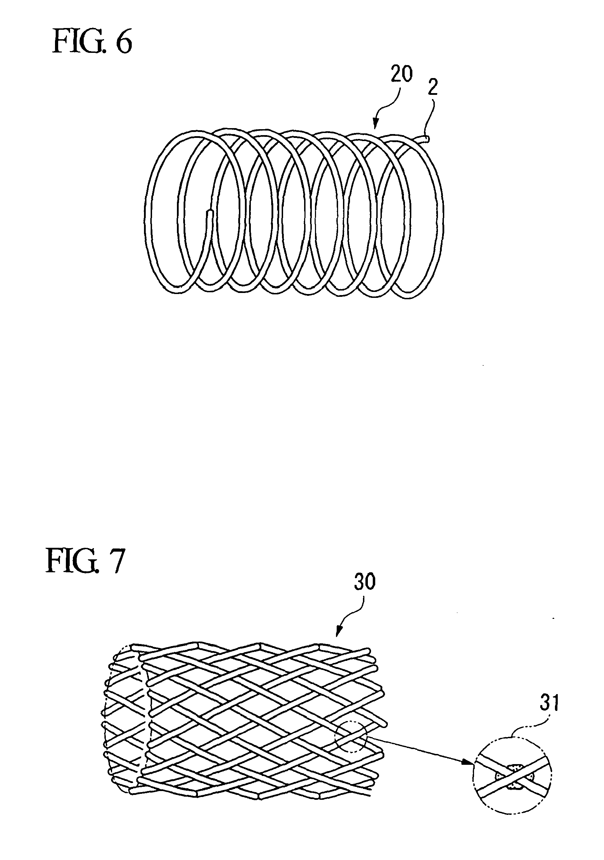

FIG 6 is a perspective view showing a second embodiment of the present invention.

Note that the same numeric symbols will be applied to structural elements that are equivalent to those of the first embodiment, and an explanation thereof will be omitted. This applies for the additional embodiments that follow below as well.

A stent 20 according to this embodiment is formed by winding a linear member 2 in which the sealed space has been filled with the UV curable resin into a helix form.

This embodiment provides the same effects as those of the first embodiment.

### <Third Embodiment>

FIG 7 is a perspective view showing a third embodiment of the present invention.

A stent 30 according to this embodiment is composed so as to form a cylindrical external shape overall, by disposing a plurality of linear members 2, in which the sealed space has been filled with the UV curable resin, into a lattice form. Intersecting areas 31 of the linear members are adhered via a suitable adhering method such as heat welding or employment of an adhesive agent.

This embodiment provides the same effects as those of the first embodiment.

### <Fourth Embodiment>

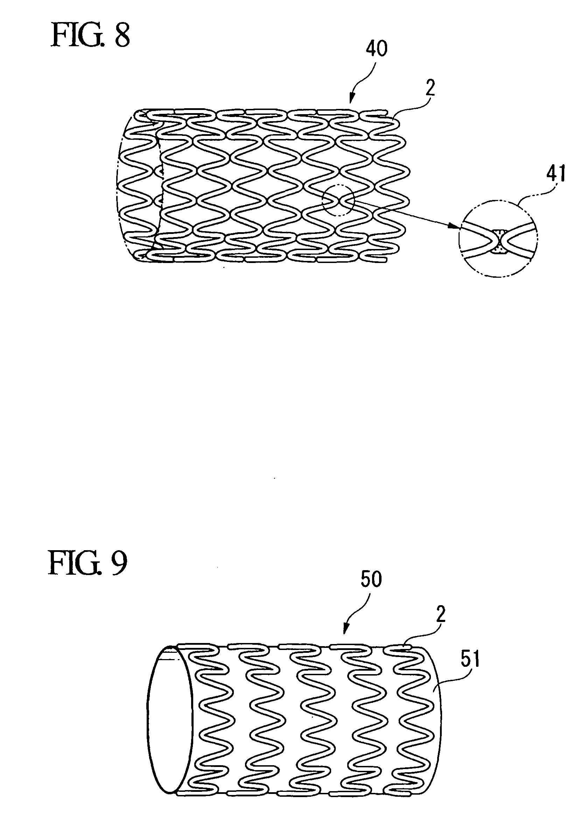

FIG 8 is a perspective view showing a fourth embodiment of the present invention.

A stent 40 according to this embodiment is composed such that the linear members 2, in which the sealed space has been filled with the UV curable resin, form a zigzag form along the longitudinal direction of the axis and so that the entire structure describes a helix. Intersecting areas 41 of the linear members 2 are adhered via a suitable adhering method such as heat welding or employment of an adhesive agent.

This embodiment provides the same effects as those of the first embodiment.

### <Fifth Embodiment>

FIG 9 is a perspective view showing a fifth embodiment of the present invention.

A stent 50 according to this embodiment is composed by adhering the linear members 2, in which the sealed space has been filled with the UV curable resin, to a surface of a flexible sheet 51 so as to form a zigzag form along the longitudinal direction of the axis and so that the entire structure describes a helix. The adhesion of the linear members 2 to the flexible sheet 51 is carried out by a suitable adhering method such as heat welding or employment of an adhesive agent. The flexible sheet 51 employs a material which is not harmful to the human body, suitable examples thereof including fluorine resins, nylon resins, urethane resins and the like. Note that when the linear members are adhered to the outside of the flexible sheet 51 as shown in FIG 9, then the flexible sheet 51 must be a material that permits transmission of UV rays so that UV rays radiated from inside can reach the inside of the linear members 2.

In addition to offering the same effects as those of the first embodiment, this embodiment enables an approximate shape to be specified through the employment of flexible sheet 51. As a result, the amount of linear members 2 employed can be reduced, and manufacture is facilitated.

### <Sixth Embodiment>

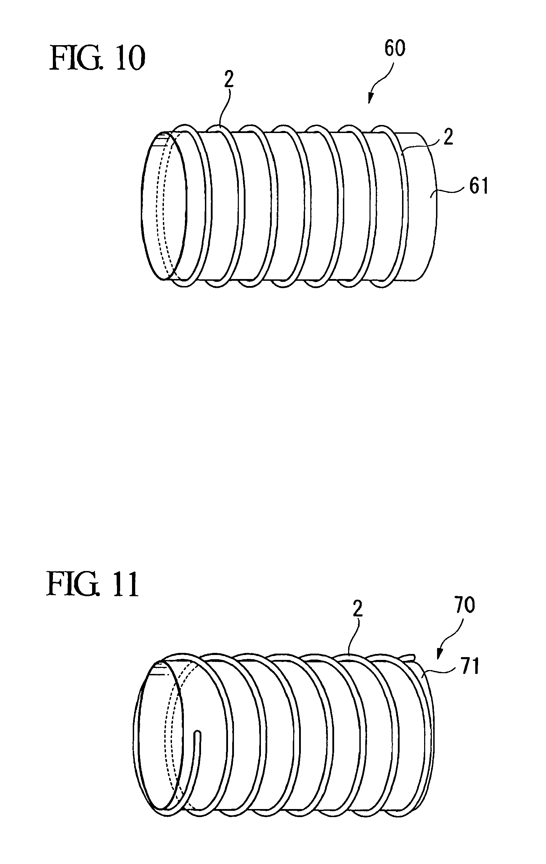

FIG 10 is a perspective view showing a sixth embodiment of the present invention.

A stent 60 according to this embodiment is composed by adhering a plurality of linear members 2, in which the sealed space has been filled with the UV curable resin, to a surface of a flexible sheet 61 in a manner so as to provide a spacing between the linear members 2 along the axial direction, and that respective linear members 2 for a ring shape.

This embodiment provides the same effects as those of the fifth embodiment.

### <Seventh Embodiment>

FIG 11 is a perspective view showing a seventh embodiment of the present invention.

A stent 70 according to this embodiment is composed by adhering a linear member 2, in which the sealed space has been filled with the UV curable resin, to a surface of a flexible sheet 71 so as to describe a helix form.

This embodiment offers the same effects as those of the fifth and sixth embodiments.

### <Eighth Embodiment>

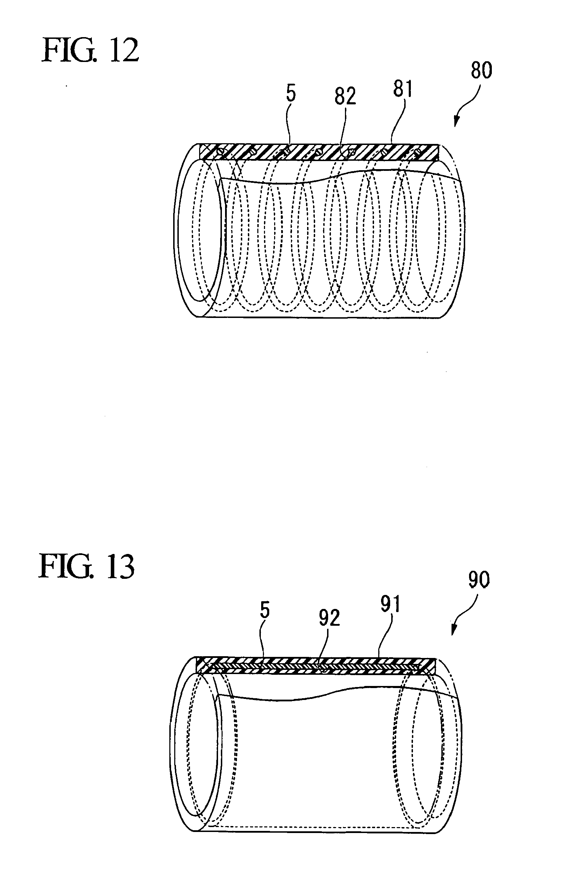

FIG 12 is a perspective view showing an eighth embodiment of the present invention.

A catheter 80 according to this embodiment is formed into a cylindrical shape in which both ends of a catheter main body 81 are open. A sealed space 82 is formed inside the lateral walls of the catheter main body 81, and this sealed space 82 is filled with the UV curable resin 5. The sealed space 82 is separated into a plurality of segments along the axial direction, with each sealed space 82 formed in the shape of a ring.

In addition to providing the same effects as those of the first embodiment, this embodiment provides the additional effect of facilitating manufacture, since the catheter 80 is directly formed without separately forming the linear members, which are an intermediate member.

### <Ninth Embodiment>

FIG 13 is a perspective view showing a ninth embodiment of the present invention.

A catheter 90 according to this embodiment is formed into a cylindrical shape in which both ends of the catheter main body 91 are open. A sealed space 92 is formed inside the lateral walls of the catheter main body 91, and this sealed space 92 is filled with the UV curable resin 5. The sealed space 92 is formed in the shape of a cylinder along the lateral walls.

This embodiment offers the same effects as those of the eighth embodiment.

Note that the present invention is not limited to the various preferred embodiments described above. Rather, suitable design modifications are possible provided that they do not depart from the spirit of the invention.

For example, while the various preceding embodiments described filling the sealed space in the medical tube with the UV curable resin, the present invention is not limited thereto. Rather, it is also acceptable to use other resins which can be cured using energy, such as a thermosetting resin which cures with the application of heat, or a resin which cures by irradiation with a light other than UV rays.

In the case of a UV curable resin, the resin becomes extremely hard if irradiated for a lengthy period of time with UV rays of a specific strength. Further, when the intensity of the UV rays is weak, or the radiation time is short, curing of the resin halts at a gel state where the hardness is only slightly increased. This may be taken advantage of to provide any degree of hardness prior to completely curing the resin, based on the amount (including duration of application) of energy provided.

The resin employed is not limited to just one type; rather a plurality of types may be employed.

Following manufacture, the stent or catheter according to the preceding embodiments may be shipped after being covered with packaging that does not transmit UV rays. As a result, the stent, etc., is covered with packaging and thus maintained in a state impervious to UV radiation during the time from manufacture until insertion into the patient's body. As a result, such undesirable events as careless exposure to UV rays leading to curing prior to insertion into the patient's body can be avoided.

The preceding embodiments employed as an example of the medical instrument, the case of a stent or catheter that is retained at a stricture site in the esophagus. However, the present invention is not limited thereto. Namely, the present invention is also applicable to other medical instruments, such as, for example, a medical use needle, medical instrument or the like.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

## Claims

1. A medical tube comprising:
a tube main body (3);
a sealed space (4) which is formed in the tube main body and is sealed on both ends; and
a variable hardness resin (5) which fills the sealed space and the hardness of which can be changed by supplying energy thereto.

2. A medical tube comprising:
a tube main body (3) having at least one lumen (4);
a variable hardness resin (5) which fills the inside of the lumen and the hardness of which can be changed by supplying energy thereto; and
an outflow preventing mechanism (6) which prevents the filling variable hardness resin from flowing out from the lumen.

3. A medical tube according to claim 1 or claim 2, wherein the variable hardness resin is an energy curable resin which cures with the addition of energy.

4. A medical instrument (1, 20, 30, 40, 50, 60) employing the medical tube according to claim 3, wherein:
a linear member (2) is formed of the medical tube with the energy curable resin filling along the central line of the tube main body; and
a cylindrical outer form of the medical instrument is formed of the linear member.

5. A medical instrument according to claim 4, wherein the cylindrical outer form is formed by interweaving the liner member.

6. A medical instrument according to claim 4, wherein the cylindrical outer form is formed by adhering the linear member to a surface of a flexible sheet (51, 61, 71).

7. A medical instrument according to claim 6, wherein the linear member is affixed to the flexible sheet in a ring or helix shape.

8. A medical instrument according to one of claims 4 through 7, wherein the tube main body is made of a UV transmitting material and the energy curable resin is made of a UV curable resin.

9. A medical instrument (80, 90) employing the medical tube according to claim 1, wherein:
a cylindrical catheter main body (81, 91) is formed such that both ends of the tube main body are open and the sealed space is formed within the side walls of the catheter main body; and
the sealed space (82, 92) is filled with the variable hardness resin.

10. A medical instrument according to one of claims 4 though 9, wherein the medical instrument is a stent.

11. A stent set employing the stent according to claim 10, wherein the stent is covered with a packaging that does not transmit UV rays.

12. An endoscope device employing the medical instrument according to claim 8, comprising:
an endoscope (10) equipped with an inserted part that incorporates the medical instrument; and
a UV ray probe (12) that can be inserted into a channel of the inserted part and which emits UV rays.
